# EUROPEAN PATENT APPLICATION

(11) **EP 4 491 240 A1**
(43) Date of publication of application: **15.01.2025**
(21) Application number: 23185045.4
(22) Date of filing: 12.07.2023
(51) Int. Cl.: A61Q 5/10, A61K 8/41

(54) **HAIR-DYEING BASE COMPOSITION COMPRISING AN IMPROVED ALKALIZING AGENT, MULTI-COMPARTMENT KIT COMPRISING THE COMPOSITION, AND METHODS OF DYEING HAIR BY USING THE SAME**

(71) Applicant: Kemon S.P.A., 06016 San Giustino (PG) (IT)
(72) Inventor: Terzino, Francesca, SAN GIUSTINO (PG) (IT); Celestini, Sabrina, SAN GIUSTINO (PG) (IT); Nocentini, Benedetta, SAN GIUSTINO (PG) (IT); Nocentini, Giuliano, SAN GIUSTINO (PG) (IT)
(74) Representative: Villa, Livia

(57) **Abstract**

A hair-dyeing base composition comprising an improved alkalizing agent is disclosed, being improved in terms of overall reduction of ammonia compounds, thus advantageously minimizing the concerns related thereto. Said composition provides a satisfactory colour hair coverage, without the malodour or harm to the scalp typically ascribable to ammonia compounds, at the same time being devoid of ingredients known to raise concerns of skin harmfulness, such as ethoxylated compounds, PEG and its derivatives, and silicones, as it includes a high percentage of natural compounds. The inventive composition is therefore significantly more healthy for the clients/consumers and hair stylists handling the same, while keeping high performance in hair-dyeing. A multi-compartment kit comprising said composition, a dye and an oxidizing component is also disclosed, as well as their use in permanently dyeing hair. Are also disclosed processes of producing said hair-dyeing base composition, as well as hair-dyeing methods encompassing the application of said composition to hair.

## Description

### FIELD OF THE INVENTION

The present invention relates to a hair-dyeing base composition comprising an improved alkalizing agent, in terms of overall reduction of ammonia compounds, thus advantageously minimizing the concerns related thereto. Indeed, the composition provides a satisfactory colour hair coverage, without the malodour or harm to the scalp typically ascribable to ammonia compounds, at the same time being devoid of monoethanolamine and other ingredients known to raise concerns of skin harmfulness, such as ethoxylated compounds, PEG and its derivatives, and silicones, as it includes a high percentage of natural compounds.

The inventive composition is therefore significantly more healthy for the clients/consumers and hair stylists handling the same, while keeping high performance in hair-dyeing.

The present invention also relates to a multi-compartment kit comprising the composition, processes of producing said hair-dyeing base composition, as well as hair-dyeing methods encompassing the application of said composition to hair.

### BACKGROUND OF THE INVENTION

Hair dyes are intended to give hair a different colour than its natural colour. Based on the duration of the colour on hair, the following dyes can distinguished: permanent dye (that lasts more than three months), semi-permanent dye (that lasts about a month and a half), gradual dye (that involves several applications to obtain the desired result), and finally the temporary dye (that lasts for a very few washes).

With particular reference to permanent dyes, the latter are known to give the desired colour through the application of chemical substances, i.e. colorants or their precursors that react with each other and especially with the hair, thus creating coloured polymers: this reaction is known as oxidative coupling between a primary intermediate colorant (such as p-phenylenediamine and p-aminophenol), and a colorant called coupler or secondary intermediate (such as 1-naphthols, m-diamines, m-aminophenols, resorcinols and pyrazolones), typically in aqueous solution with oxidizing agents, such as hydrogen peroxide.

Among the advantages of permanent dyes are good coverage of grey hair and a wide range of colours, while the disadvantages are the possible and frequent allergies they cause.

Actually, the percentage of colouring substances present in permanent dyes usually does not exceed 6% to avoid product toxicity.

Permanent dyes are thus oxidative-type dyes, as formed *in situ* by oxidative coupling of a substance defined as a primary intermediate or oxidation base and a substance known as a coupler or secondary intermediate in the presence of an oxidizing agent. The oxidation colouring process involves the direct application of a dye or its precursors, or a primary intermediate and a coupler mixed with an aqueous solution of hydrogen peroxide as an oxidizing agent, this mixture is then left to fix on the fibres for a determined time and subsequently it is removed by rinsing, once the fibres have become coloured.

Components for suitable oxidative-type dyes, which must be applied to the hair and in contact with the scalp, must meet very stringent requirements.

First of all, they must develop a sufficient intensity of colouring in the desired shade, furthermore this colouring must be resistant to external agents, such as adverse weather conditions, frequent hair washing, styling and perspiration. The dyes must also be compliant from a toxicological and dermatological point of view and must not cause sensitisation.

The components present in permanent oxidative dyes are mainly colouring substances, the aforementioned couplers and oxidizing agents, substances useful for maintaining a basic pH, such as ammonia or monoethanolamine, also named alkalizing substances.

With respect to the latter, A.D. Bailey et al., 2014 ["Comparison of damage to human hair fibers caused by monoethanolamine and ammonia based hair colorants", Journal of Cosmetic Science 65(1): 1-9] have reported that (see Abstract) "The number of Level 3 hair color products that substitute 2-aminoethanol [monoethanolamine (MEA)] for ammonia is increasing. There is some anecdotal evidence that higher levels of MEA can be more damaging to hair and more irritating than a corresponding equivalent level of the typical alkalizer, ammonia (in the form of ammonium hydroxide). Our interest was to understand in more quantitative terms the relative hair damage from the two alkalizers, particularly at the upper limits of MEA on-head use. Limiting investigations of oxidative hair damage to increases in cysteic acid content (from cystine oxidation) can underreport the extent of total damage. Hence, we complemented Fourier transform infrared spectroscopy (FTIR) cysteic acid level measurement with scanning electron microscopy (SEM) photomicrographs to visualize cuticle damage, and protein loss to understand not only the oxidative damage but also the damage caused by other damage pathways, e.g., reaction of the more nucleophilic (than ammonia) MEA with hair protein. In fact, all methods show an increase in damage from MEA-based formulations, up to 85% versus ammonia in the most extreme case. Hence, if the odor of ammonia is a concern, a better approach may be to minimize the volatility of ammonia in specific chassis rather than replacing it with high levels of a potentially more damaging alkalizer such as MEA."

Therefore, the object of the present invention is to provide a further improved hair-dying base, that is effective as, or even more than, the aforementioned products of the prior art, and which, at the same time, overcomes the limitations of the same, especially with reference to the alkalizing substances.

### SUMMARY OF THE INVENTION

The above object has been achieved by a hair-dyeing base composition comprising an improved alkalizing agent, as set forth by the present claims.

In particular, said improved alkalizing agent allows the composition to minimize the concerns related to ammonia compounds, such as the malodour or harm to the scalp typically ascribable to ammonia compounds, while providing a satisfactory colour hair coverage and minimizing damage to the fibres compared to commercial or conventional hair colour compositions; at the same time the composition is devoid of ingredients known to raise concerns of skin harmfulness, such as ethoxylated compounds, PEG and its derivatives, and silicones, as it includes a high percentage of natural compounds.

The hair colour base composition of this invention is also appreciably durable and wash/fade resistant.

In another aspect, the present invention also relates to a process of producing said hair-dyeing base composition.

In a further aspect, the present invention also relates to hair-dyeing methods encompassing the application of said composition to hair.

In an additional aspect, the present invention concerns a multi-compartment kit for dyeing hair, the kit comprising a first compartment A) comprising the hair-dyeing base composition, and a dye, and a second compartment B) comprising the oxidizing component.

In a further aspect, the present invention concerns the use of the hair-dyeing base composition for permanently dyeing hair.

### BRIEF DRESCTIPTION OF THE DRAWINGS

The characteristics and advantages of the present invention will be evident from the detailed description given below, from the illustrative non-limiting working examples, as well as from the annexed figures, wherein:

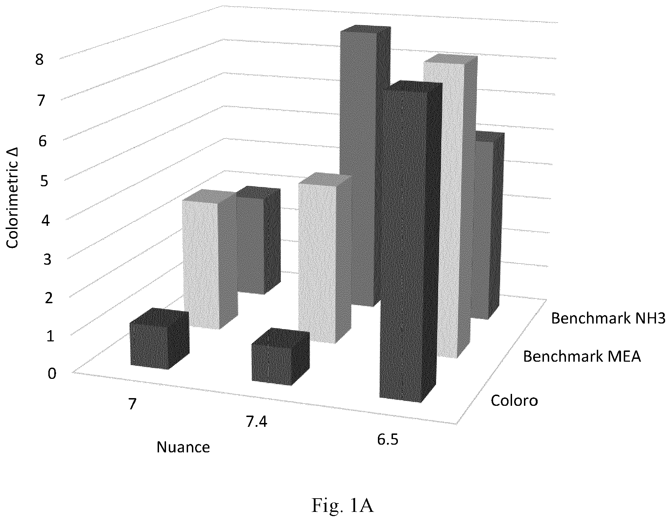

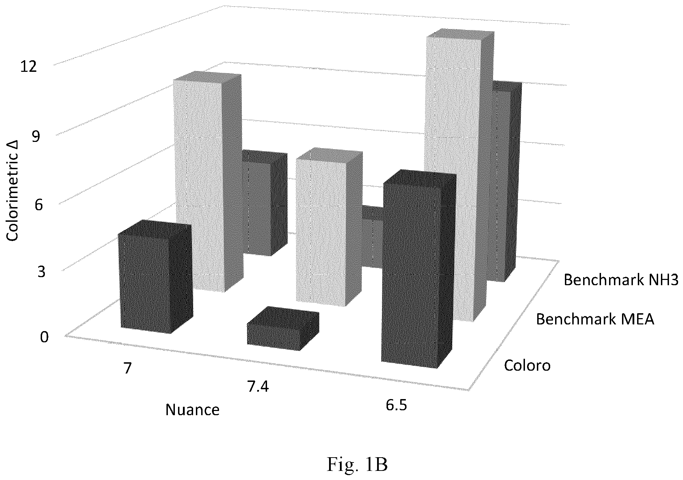

Figures 1A and 1B show the results of colour retention tests after washing, as performed in Example 6 on locks of yak hair (*), and particularly:
   Figure 1A shows the results of colour retention tests after 10 washing cycles with a standard post-colouring shampoo, wherein the locks coloured with Nuance 7, Nuance 7.4 or Nuance 6.5, in the hair-dyeing base composition of the invention (shortly referred to as "Coloro") were compared to locks coloured with Nuance 7, Nuance 7.4 or Nuance 6.5, in a comparative MonoEthanolAmine-based hair-dyeing composition (shortly referred to as "Benchmark MEA") and coloured with Nuance 7, Nuance 7.4 or Nuance 6.5, in a comparative ammonia-based hair-dyeing composition (shortly referred to as "Benchmark NH3"); and
   Figure 1B shows the results of colour retention tests after 10 washing cycles with a standard intensive treatment shampoo (pH 8), wherein the locks coloured with "Coloro" were compared to locks coloured with "Benchmark MEA" and coloured with "Benchmark NH3";

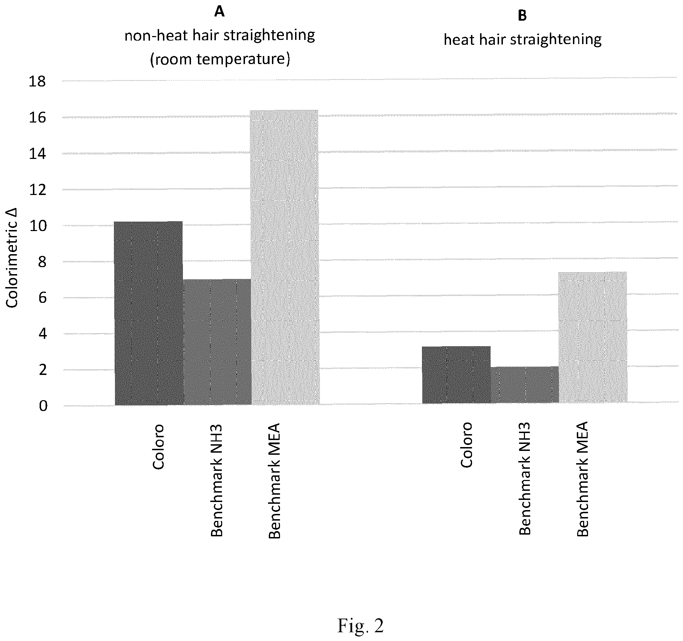

Figure 2 shows the results of decolouration of dyed hair by using a decolourant product, as performed in Example 7 on locks of yak hair, with respect to the starting decoloured locks, wherein the decolouration of locks previously coloured with "Coloro" were compared to the decolouration of locks previously coloured with "Benchmark MEA" and previously coloured with "Benchmark NH3", under two different conditions, i.e. after non-heat straightening (A) and after heat straightening (B);

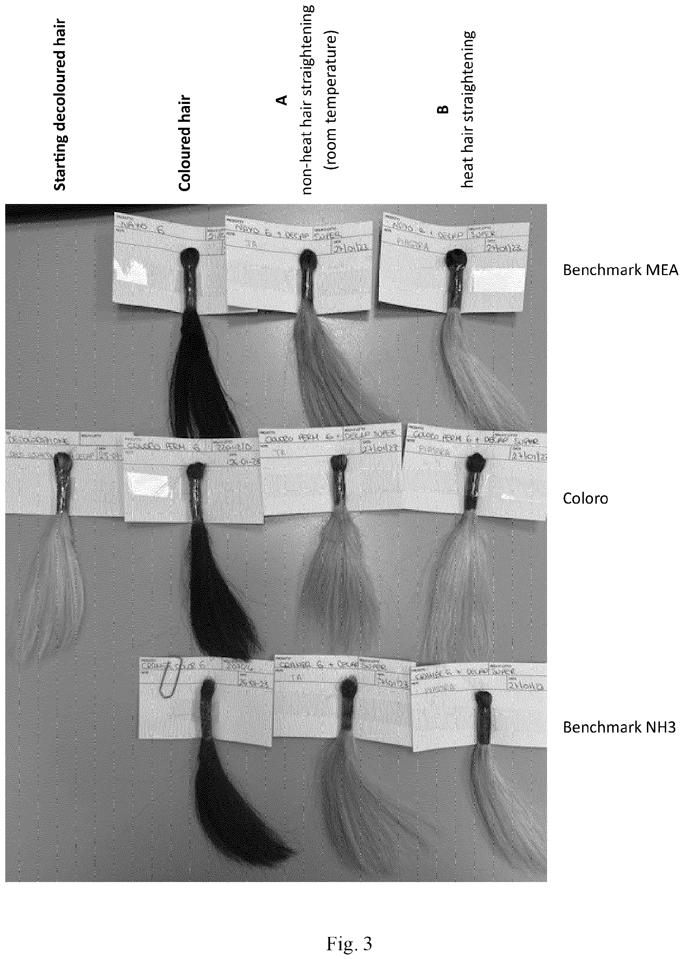

Figure 3 is a picture of the samples tested in Example 7 and graphically depicted in Figure 2;

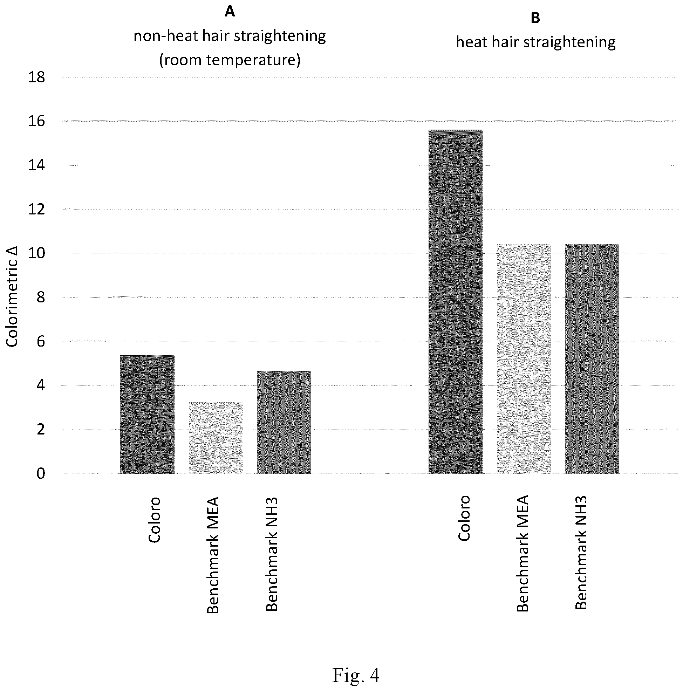

Figure 4 shows the results of lightening of dyed hair by applying a further dye (i.e. Nuance 10), as performed in Example 8 on locks of yak hair, wherein the lightening of dyed locks further coloured with "Coloro" (i.e. the hair-dyeing base composition of the invention + Nuance 10) were compared to the lightening of dyed locks further coloured with "Benchmark MEA" (i.e. a comparative MEA-based hair-dyeing composition + Nuance 10), and dyed locks further coloured with "Benchmark NH3" (i.e. a comparative NH3-based hair-dyeing composition + Nuance 10), under two different conditions, i.e. after non-heat straightening (A) and after heat straightening (B);

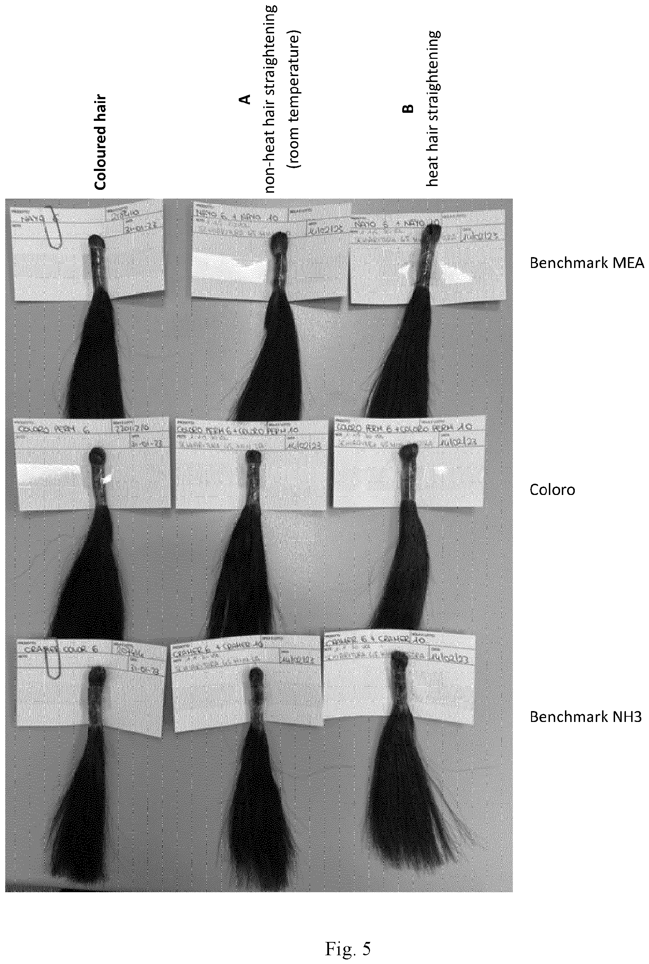

Figure 5 is a picture of the samples tested in Example 8 and graphically depicted in Figure 4;

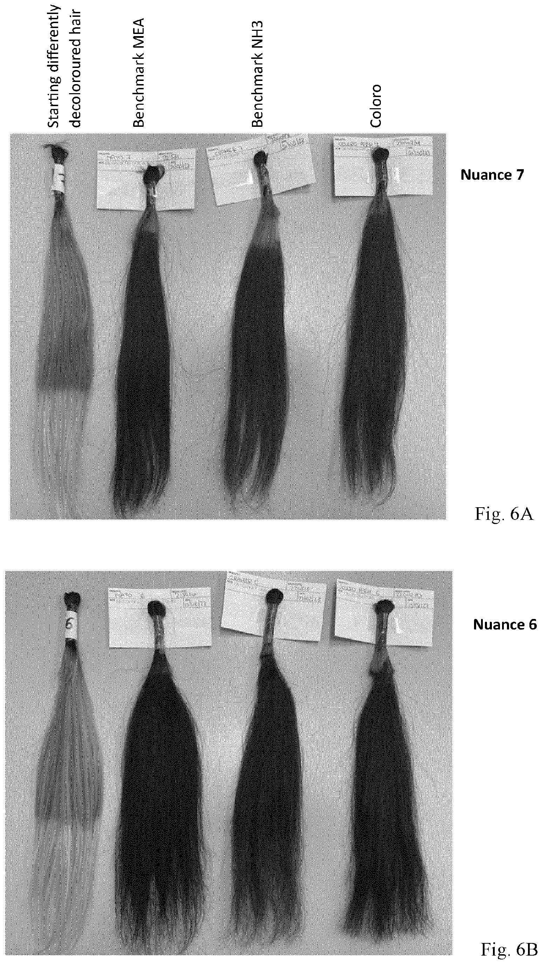

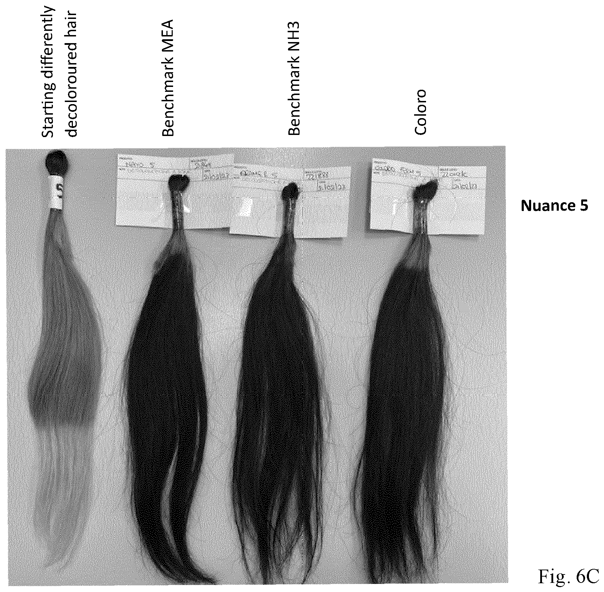

Figures 6A, 6B and 6C are pictures showing the results of the colouring coverage of decoloured yak locks after application of a dye, as performed in Example 9, wherein the starting locks were subjected to a first step of decolouration, and then subjected to a second step of decolouration but only on the hair ends; particularly, said dye was Nuance 7 in Figure 6A, Nuance 6 in Figure 6B, and Nuance 5 in Figure 6C; for each nuance, the three different hair-dying base compositions "Coloro", "Benchmark MEA" and "Benchmark NH3", were tested.

(^{∗}) Yak belly hair are currently widely used as a cheap substitute for human hair for the development of hair dyes, as its chemical composition closely resembles human hair in Raman spectroscopy. The absence of melanin in yak belly hair also leads to a strong reduction of fluorescence in Raman measurements, which is advantageous for the investigation of the effectivity of hair dyes.

### DETAILED DESCRIPTION OF THE INVENTION

The present invention therefore relates to a hair-dyeing base composition comprising an alkalizing agent, said alkalizing agent comprising 0.01-10.00wt% of dimethylglucamine and 0.01-2.00wt% of ammonia, based on the composition weight, and being free of monoethanolamine.

It has being surprisingly found that when a hair-dyeing base is alkalinized in presence of dimethylglucamine, it is possible to advantageously avoid the use of monoethanolamine, and at the same time reduce the overall content of ammonia to a percentage not higher than 2.00wt%. This results in a hair-dyeing base composition which is suitably stable at basic pH, and which has advantageously minimized the release of unpleasant odour deriving from ammonia, while anyway taking benefit of the presence of the latter in the composition. Dimethylglucamine (CAS No.: 76326-99-3), also known as N,N-Dimethylglucamine, 1-Deoxy-1-(dimethylamino)-D-glucitol, (2R,3R,4R,SS)-6-(Dimethylamino)hexane-1,2,3,4,5-pentaol, (2R,3R,4R,5S)-6-(dimethylamino)hexane-1,2,3,4,5-pentol, or methylmeglumine, is a compound typically used as a reagent in the synthesis of N,N-dimethyl-N-alkyl-D-glucaminium bromides (which exhibit antimicrobial activity). Therefore, its direct use in hair dying products is unprecedented, much more with reference to its role in effectively substituting monoethanolamine and complementing ammonia action.

Preferably, the weight ratio between dimethylglucamine and ammonia is 1:1 to 1:5, more preferably 1:1.1 to 1:3.5, even more preferably 1:1.2 to 1:2.5.

More preferably, the alkalizing agent of the inventive composition comprises 0.05-5.00wt% of dimethylglucamine and 0.30-1.90wt% of ammonia, based on the composition weight.

Even more preferably, the alkalizing agent of the inventive composition comprises 0.50-2.00wt% of dimethylglucamine and 0.50-1.85wt% of ammonia, based on the composition weight.

In preferred embodiments, said alkalizing agent further comprises aminomethyl propanol, ammonium bicarbonate, or a mixture thereof.

More preferably, said alkalizing agent comprises a mixture of 0.01-3.00wt% of aminomethyl propanol, 0.01-2.50wt% of ammonium bicarbonate, based on the composition weight. Even more preferably, said alkalizing agent comprises a mixture of 0.10-2.90wt% of aminomethyl propanol, 0.10-2.00wt% of ammonium bicarbonate, based on the composition weight.

In particularly preferred embodiments, said alkalizing agent comprises a mixture of 0.95-1.90wt% of dimethylglucamine, 1.00-1.80wt% of ammonia, 0.50-2.85wt% of aminomethyl propanol, and 0.50-1.50wt% of ammonium bicarbonate, based on the composition weight.

In most preferred embodiments, said alkalizing agent consists of a mixture of dimethylglucamine, aminomethyl propanol, ammonia, and ammonium bicarbonate. It has been observed that the mixture of these four compounds in the composition of the invention allows to improve the performance on lightening of dyed hair, when subsequently coloured with the inventive composition.

The hair-dyeing base composition of the invention also comprises water, at least a primary intermediate, at least a secondary intermediate, at least an antioxidant, a lipophilic matrix, and a hydrophilic matrix.

The hair-dyeing base composition of the invention is also advantageously free of ethoxylated compounds, polyethylene glycols, sulphates, and silicones. In this regard, a satisfactory and highly performing composition has been achieved by using substitutive ingredients having a natural origin, such as coconut oil, propanediol, sodium cocoyl glutamate, and olive oil polyglyceryl-6 esters.

The hair-dyeing base composition of the invention also comprises at least a primary intermediate preferably selected from p-phenylenediamine, toluene-2,5-diamine sulfate, p-aminophenol, n-bis(2-hydroxyethyl)-p-phenylenediamine, 4-amino-m-cresol, p-methylaminophenol sulfate, 1-hydroxyethyl 4,5-diamino pyrazole sulfate, and mixtures thereof.

More preferably, said at least a primary intermediate is selected from toluene-2,5-diamine sulfate, p-aminophenol, p-methylaminophenol sulfate, and mixtures thereof.

In preferred embodiments, the hair-dyeing base composition of the invention comprises 0.01-5.00wt% of said at least a primary intermediate, based on the composition weight.

In more preferred embodiments, the hair-dyeing base composition of the invention comprises 0.02-3.00wt% of said at least a primary intermediate, based on the composition weight.

The hair-dyeing base composition of the invention also comprises at least a secondary intermediate preferably selected from resorcinol, m-aminophenol, 1-naphthol, 4-chlororesorcinol, 4-amino-2-hydroxytoluene, 2-methylresorcinol, phenyl methyl pyrazolone, 2-amino-4-hydroxyethylaminoanisole sulfate, 2-amino-3-hydroxypyridine, 2,4-diaminophenoxyethanol sulfate, 2-methyl-5-hydroxyethylaminophenol, 6-amino-m-cresol, 5-amino-6-chloro-o-cresol, 2-amino-6-chloro-4-nitrophenol, 2,6-dihydroxyethylamino toluene, hydroxyethyl-3,4-methylenedioxyaniline HCl, 2,4-diaminophenoxyethanol HCl, and mixtures thereof.

More preferably, said at least a secondary intermediate is selected from m-aminophenol, 4-chlororesorcinol, 2-methylresorcinol, phenyl methyl pyrazolone, 2,4-diaminophenoxyethanol HCl, and mixtures thereof.

In preferred embodiments, the hair-dyeing base composition of the invention comprises 0.01-5.00wt% of said at least a secondary intermediate, based on the composition weight.

In more preferred embodiments, the hair-dyeing base composition of the invention comprises 0.05-4.00wt% of said at least a secondary intermediate, based on the composition weight.

In other embodiments, the weight ratio between said at least a primary intermediate and said at least a secondary intermediate is 1:1 to 1:3.

Compositions according to the invention may optionally comprise one or more synthetic or natural direct dyes, selected from anionic, cationic and nonionic species, and mixtures thereof. Examples of suitable direct dyes may include Basic Red 51, Basic Orange 31, and Basic Yellow 87. Exemplary natural direct dyescan be lawsone, juglone, alizarin, purpurin, indigo, isatin, curcumin and mixtures thereof. Extracts or decotions containing these natural hair dyes such as henna-based extracts may also be used.

Preferably, the hair-dyeing base composition of the invention also comprises at least an antioxidant, preferably selected from erythorbic acid, sodium hydrosulphite, sodium sulphite ascorbic acid, and mixtures thereof.

Preferably, the hair-dyeing base composition of the invention also comprises a lipophilic matrix, preferably comprising sorbitan stearate, oleic acid, petrolatum, hydrogenated castor oil, Copernicia cerifera cera, ricinus communis seed oil, oleth-4 phosphate, oleyl phosphate, laureth-3, cetearyl olivate, sorbitan olivate, cetearyl alcohol, ceteareth-20, polysorbate 60, dicaprylyl ether, stearic acid, glyceryl stearate, oleyl alcohol, sorbitan oleate, palmitic acid, olive oil polyglyceryl-6 esters, glyceryl isostearate, cera alba, BHT, crambe abyssinica seed oil, cocos nucifera oil, and mixtures thereof.

More preferably, said lipophilic matrix comprises sorbitan stearate, oleic acid, hydrogenated castor oil, Copernicia cerifera cera, ricinus communis seed oil, dicaprylyl ether, sorbitan oleate, cetearyl alcohol, hydrogenated castor oil, stearic acid, palmitic acid, olive oil polyglyceryl-6 esters, glyceryl isostearate, cocos nucifera oil, and mixtures thereof.

In preferred embodiments, the hair-dyeing base composition of the invention comprises 15-60wt% of said lipophilic matrix, based on the composition weight, more preferably 25-45wt%, even more preferably 30-40wt%.

Preferably, the hair-dyeing base composition of the invention also comprises a hydrophilic matrix, preferably comprising betaine, etidronic acid, propanediol, disodium EDTA, trisodium dicarboxymethyl alaninate, sodium cocoyl glutamate, sodium stearoyl glutamate, cocamidopropyl betaine, and mixtures thereof.

In preferred embodiments, the hair-dyeing base composition of the invention comprises 10-30wt% of said hydrophilic matrix, based on the composition weight, more preferably 15-20wt%.

In other embodiments, the weight ratio between said lipophilic matrix and said hydrophilic matrix is 3:1 to 1:1, more preferably about 2: 1.

Optionally, the hair-dyeing base composition of the invention can comprise also a parfum, preferably 0.50-2.50wt%, based on the composition weight.

Additional and optional ingredients can include vegetal oils, protein hydrolyzates, plant extracts, organic and inorganic pigments, and mixtures thereof.

In other preferred embodiments, the present invention concerns a hair-dyeing base composition comprises an alkalizing agent, said alkalizing agent consisting essentially of 0.05-3.00wt% of dimethylglucamine, 0.01-2.00wt% of ammonia, aminomethyl propanol, and ammonium bicarbonate, based on the composition weight, and being free of monoethanolamine. The term "consisting essentially of" means that the ingredients listed are the only active having alkalizing properties in the alkalizing agent, the remainder optional ingredients being solvents, such as water, of merely co-formulants having no influence on pH.

In further preferred embodiments, the present invention concerns a hair-dyeing base composition comprises an alkalizing agent, said alkalizing agent consisting of water, 0.05-3.00wt% of dimethylglucamine, 0.01-2.00wt% of ammonia, aminomethyl propanol, and ammonium bicarbonate, based on the composition weight, and being free of monoethanolamine.

It is to be understood that all aspects identified as preferred and advantageous for the composition of the invention, are to be deemed analogously preferred and advantageous also for the composition of the invention of these preferred embodiments denoted by the terms "consisting essentially of" and "consisting of'.

In another aspect, the present invention also relates to a multi-compartment kit for dyeing hair, the kit comprising a first compartment A) comprising the hair-dyeing base composition as above described, and a dye, and a second compartment B) comprising an oxidizing component.

For the purposes of the present invention, with the term "dye" is meant a colourant for hair, or its precursors, which is typically associated to numbers. Tone height numbers are unique codes that explain the many different colours and tones of hair dyes. These numbers are the same worldwide and has the structure "x.y". The first number "x", the number on the left side of the dot, is the tone height number. The higher the number, the lighter the colour. The tone heights range from 1 (black) to 10 (light blonde). The second number "y", the one on the right side of the dot, stands for the colour nuance. For example, the 1 in 7.1 stands for ash. This means it's a matte, cool, dark brown colour. Some colours have a third number "z", referring to the hue of the colour. For instance, in 4.13, the 1 stands for ash and the 3 for gold.

The dye should be mixed with an oxidizing agent at a certain percentage ratio, such as from 1:1 to 1:10, preferably 1:1 to 1:4.

The oxidizing component of the kit should contain at least one oxidizing agent, preferably selected from peroxides, persulphates, percarbonates, alkali metal bromates, ferricyanides, peroxygenated salts, and mixtures thereof.

In preferred embodiments, the oxidizing agent is hydrogen peroxide, the title of which preferably ranges from 1 to 40 volumes.

The oxidizing component of the kit may also contain solvents (such as water or organic solvents or a mixture thereof), rheological or viscosity additives, chelating agents, surfactants, pH regulators, preservatives, and/or perfume.

The oxidizing component can be in the form of powder, gel, liquid, foam, lotion, cream, mousse, or emulsion.

In another aspect, the present invention also relates to a process of producing said hair-dyeing base composition. In particular, said process comprises the steps of:
a) preparing a hydrophilic phase, by mixing hydrophilic ingredients and the alkalizing agent of the invention, at 70-75°C;
b) preparing a lipophilic phase, by mixing lipophilic ingredients at 70-75°C;
c) adding the lipophilic phase to the hydrophilic phase, under stirring, until a homogenous emulsion is obtained; and
d) the emulsion is kept under stirring for a few minutes and then cooled below 50°C, thus achieving the hair-dyeing base composition.

In preferred embodiments, wherein the said alkalizing agent further comprises aminomethyl propanol, ammonium bicarbonate, or a mixture thereof, the latter are kept aside and added after the step d) is completed, then letting the composition's temperature to raise at room temperature.

In a further aspect, the present invention relates to the use of the hair-dyeing base composition as above described, or use of the multi-compartment kit as above described, for permanently dyeing hair.

Also, the present invention also relates to hair-dyeing methods encompassing the application of said composition to hair. In particular, said method comprises the steps of:
i) mixing the hair-dyeing base composition of the invention with a dye and an oxidizing agent, until a homogeneous cream is obtained;
ii) applying the cream to hair; and
iii) rinsing with water and washing with a post-colouring shampoo.

Preferably, in step ii), the cream is left on hair for 20-50 minutes, before rinsing with water, depending on the colouring/lightening effect to achieve.

It is to be understood that all aspects identified as preferred and advantageous for the composition of the invention, are to be deemed analogously preferred and advantageous also for the kit, and both the uses and methods of application of said composition.

It is also to be understood that all the combinations of preferred and advantageous features of the composition, kit, uses and methods are deemed to be hereby described.

Working examples of the present invention provided for illustrative purposes are reported herein below.

### EXAMPLES

### Example 1

The following hair-dyeing base composition was prepared:

| **Ingredients** | **wt%** |
|---|---|
| **Primary intermediates:** | |
| toluene-2,5-diamine sulfate | 0.9108 |
| p-aminophenol | 0.0864 |
| p-methylaminophenol sulfate | 0.0279 |

| **Secondary intermediates:** | |
|---|---|
| m-aminophenol | 0.0787 |
| 4-chlororesorcinol | 0.2790 |
| 2-methylresorcinol | 0.2250 |
| phenyl methyl pyrazolone | 0.1100 |
| 2,4-diaminophenoxyethanol HCl | 0.0102 |

| **Antioxidants:** | |
|---|---|
| erythorbic acid | 0.1500 |
| sodium hydrosulfite | 0.2000 |
| sodium sulfite | 0.2000 |

| **Parfum** | |
|---|---|
| Parfum | 1.9000 |

| **Lipophilic Matrix:** | |
|---|---|
| sorbitan stearate | 4.0000 |
| oleic acid | 2.0100 |
| hydrogenated castor oil, copernicia cerifera cera, ricinus communis seed oil | 7.4600 |
| dicaprylyl ether | 0.5000 |
| sorbitan oleate | 2.0000 |
| cetearyl alcohol, sorbitan oleate, hydrogenated castor oil, stearic acid, palmitic acid | 16.2500 |
| olive oil polyglyceryl-6 esters | 0.2000 |
| glyceryl isostearate | 1.9300 |
| cocos nucifera oil | 3.0000 |

| **Hydrophilic Matrix:** | |
|---|---|
| betaine | 3.0000 |
| etidronic acid | 0.1000 |
| propanediol | 3.0000 |
| Trisodium Dicarboxymethyl Alaninate | 0.2000 |
| sodium cocoyl glutamate (42%), water | 6.0000 |
| cocamidopropyl betaine | 6.5000 |

| **Alkalinizing Agent:** | |
|---|---|
| aminomethyl propanol (95%), water | 1.0000 |
| dimethylglucamine (50%), water | 2.0000 |
| ammonia (30%), water | 4.5000 |
| ammonium bicarbonate | 1.0000 |

| **Water** | 31.1700 |
|---|---|
| | |
| Total | 100.0000 |

In the present case, the actual concentration of dimethylglucamine was 1wt%, based on the composition weight, whereas the actual concentration of ammonia was 1.35wt%, thus very low.

The first step was the preparation of the hydrophilic phase. The ingredients were mixed, heated to 70-75°C, the oxidation intermediates, the alkalizers except ammonium bicarbonate and the antioxidants are added.

In the second step, the lipophilic phase was heated to 70-75°C and the lipophilic phase was added to the hydrophilic phase under stirring to form the emulsion. The mixture was kept under stirring for a few minutes and then cooled below 50°C. Ammonium bicarbonate, active ingredients and perfume were added and the composition let to reach room temperature.

### Example 2

The following hair-dyeing base composition was prepared:

| **Ingredients** | **wt%** |
|---|---|
| **Primary intermediates:** | |
| toluene-2,5-diamine sulfate | 1.0120 |
| p-aminophenol | 0.0960 |
| p-methylaminophenol sulfate | 0.0310 |

| **Secondary intermediates:** | |
|---|---|
| m-aminophenol | 0.0875 |
| 4-chlororesorcinol | 0.3100 |
| 2-methylresorcinol | 0.2500 |
| phenyl methyl pyrazolone | 0.1230 |
| 2,4-diaminophenoxyethanol HCl | 0.0114 |

| **Antioxidants:** | |
|---|---|
| erythorbic acid | 0.1500 |
| sodium hydrosulfite | 0.2000 |
| sodium sulfite | 0.2000 |

| **Parfum** | |
|---|---|
| Parfum | 1.9000 |

| **Lipophilic Matrix:** | |
|---|---|
| sorbitan stearate | 4.0000 |
| oleic acid | 2.0100 |
| hydrogenated castor oil, copernicia cerifera cera, ricinus communis seed oil | 7.4600 |
| dicaprylyl ether | 0.5000 |
| sorbitan oleate | 2.0000 |
| cetearyl alcohol, sorbitan oleate, hydrogenated castor oil, stearic acid, palmitic acid | 16.2500 |
| olive oil polyglyceryl-6 esters | 0.2000 |
| glyceryl isostearate | 1.9300 |
| cocos nucifera oil | 3.0000 |

| **Hydrophilic Matrix:** | |
|---|---|
| betaine | 3.0000 |
| etidronic acid | 0.1000 |
| propanediol | 3.0000 |
| Trisodium Dicarboxymethyl Alaninate | 0.2000 |
| sodium cocoyl glutamate (42%), water | 6.0000 |
| cocamidopropyl betaine | 6.5000 |

| **Alkalinizing Agent:** | |
|---|---|
| aminomethyl propanol (95%), water | 1.0000 |
| dimethylglucamine (50%), water | 2.5000 |
| ammonia (30%), water | 4.0000 |
| ammonium bicarbonate | 1.0000 |
| **Water** | 31.1700 |
| | |
| Total | 100.0000 |

In the present case, the actual concentration of dimethylglucamine was 1.25wt%, based on the composition weight, whereas the actual concentration of ammonia was 1.20wt%, thus even lower than Example 1.

The preparation steps were the same as per Example 1.

### Examples 3-5

The composition of Example 1 was prepared, while having changed only the alkalinizing agent (and the overall content of water as balance to 100wt%), and having kept unchanged the other ingredients.

The following alkalinizing agents have been used:

| **Alkalinizing Agent:** | Ex. 3 | Comp. Ex. 4 | Comp. Ex. 5 |
|---|---|---|---|
| aminomethyl propanol (95%), water | 0.0000 | 0.0000 | 0.0000 |
| dimethylglucamine (50%), water | 2.0000 (1.0000) | 0.0000 | 0.0000 |
| ammonia (30%), water | 4.5000 (1.3500) | 7.5000 (2.2500) | 0.0000 |
| monoethanolamine | 0.0000 | 0.0000 | 7.5000 |
| ammonium bicarbonate | 0.0000 | 0.0000 | 0.0000 |

The value between brackets denotes the actual concentration of the ingredient in the corresponding composition.

The composition of Example 3, according to the present invention, includes dimethylglucamine and ammonia.

In the comparative composition of Example 4, ammonia was used as the only alkalinizing agent.

In the comparative composition of Example 5, monoethanolamine was used instead of ammonia, as the only alkalinizing agent.

Herein below, the composition of Example 3 is referred to as "Coloro", the comparative composition of Example 4 is referred to as "Benchmark NH3", whereas the comparative composition of Example 5 is referred to as "Benchmark MEA".

### Example 6 - Colour retention tests after washing

An overall number of 90 locks of yak hair were divided into three groups and then dyed with:
Group I: locks coloured with Nuance 7, Nuance 7.4 or Nuance 6.5, in the hair-dyeing base composition of Example 3 ("Coloro")
Group II: locks coloured with Nuance 7, Nuance 7.4 or Nuance 6.5, in the comparative MonoEthanolAmine-based hair-dyeing composition of Example 4 ("Benchmark NH3")
Group III: locks coloured with Nuance 7, Nuance 7.4 or Nuance 6.5, in the comparative ammonia-based hair-dyeing composition of Example 5 (shortly referred to as "Benchmark MEA")

Each group of so dyed locks was sub-divided in three sub-groups, for assessing the colour retention after washing with shampoo, as follows:
Sub-groups CTRL: control sub-group, not washed
Sub-groups A: locks subjected to 10 washing cycles with a standard post-colouring shampoo
Sub-groups B: locks subjected to 10 washing cycles with intensive treatment shampoo (pH 8)

Figures 1A and 1B show the results of colour retention tests after washing, as above reported. In particular, Figure 1A shows the results of colour retention tests of sub-groups A, wherein the locks coming from Group I ("Coloro") have shown a better colour retention, as measured via colorimeter (i.e. spectrophotometer Konica Minolta CM-3600A), as well as visually, with respect to corresponding sub-group CTRL, than the locks coming from Group II ("Benchmark NH3") and Group III ("Benchmark MEA")

Figure 1B shows the results of colour retention tests of sub-groups B, wherein again the locks coming from Group I ("Coloro") have shown a better colour retention, as measured via colorimeter, as well as visually, with respect to corresponding sub-group CTRL, than the locks coming from Group II ("Benchmark NH3") and Group III ("Benchmark MEA")

These results confirm that the hair-dyeing base composition comprising the inventive alkalinizing agent, allows to achieve a satisfactory long-lasting persistence of the dye on hair, which is improved with respect to the comparative compositions.

### Example 7 - Decolouration of dyed hair by using a decolourant product

A starting overall number of 90 decoloured locks of yak hair were divided into three groups, and then dyed with Nuance 10 in:
Group I: "Coloro"
Group II: "Benchmark NH3"
Group III: "Benchmark MEA"

A starting decoloured lock of yak hair is illustrated in Figure 3, as "starting decoloured hair", while the dyed locks of the three groups above are illustrated, under "coloured hair".

The dyed locks of all the three groups were then subjected to a double cycle of decolouration using a standard decolouring product. After that, the locks were sub-divided into two sub-groups for each group:
Sub-groups A: locks subjected to non-heat hair straightening (i.e. room temperature)
Sub-groups B: locks subjected to heat hair straightening

The resulting straightened locks are illustrated in Figure 3, pictures on the right.

Figure 2 graphically shows the results of decolouration of dyed hair by using a decolourant product, with respect to the starting decoloured locks, wherein the double decolouration of locks of group "Coloro" were compared to the double decolouration of locks of group "Benchmark MEA" and "Benchmark NH3", under two different conditions, i.e. after non-heat straightening (A) and after heat straightening (B).

Again, the locks treated with Coloro have shown better results in terms of minimized delta between the starting lock and double decoloured locks, as measured by colorimeter and visually assessed. Actually, in this case, the lower the difference (delta) between the starting decoloured lock and the final double decoloured locks, the better the performance result obtained.

### Example 8 - Lightening of dyed hair by applying a further dye

A starting overall number of 90 locks of yak hair coloured with Nuance 6, were divided into three groups, and then lightened by applying a further dye, i.e. Nuance 10 in:
Group I: "Coloro"
Group II: "Benchmark NH3"
Group III: "Benchmark MEA"

A starting coloured lock of yak hair is illustrated in Figure 5, as "coloured hair", while the lightened locks of the three groups above are illustrated, and further sub-divided into two sub-groups for each group:
Sub-groups A: locks subjected to non-heat hair straightening (i.e. room temperature)
Sub-groups B: locks subjected to heat hair straightening

The aim of this test was to investigate the possibility of lighten the initial intense brown colour (Nuance 6) with a further coloration step involving the Nuance 10.

As reported in Figure 4, the results of lightening of dyed hair by applying a further dye (i.e. Nuance 10), on locks of yak hair, wherein the lightening of dyed locks further coloured with "Coloro" (i.e. the hair-dyeing base composition of the invention + Nuance 10) were compared to the lightening of dyed locks further coloured with "Benchmark MEA" (i.e. a comparative MEA-based hair-dyeing composition + Nuance 10), and dyed locks further coloured with "Benchmark NH3" (i.e. a comparative NH3-based hair-dyeing composition + Nuance 10), under two different conditions, i.e. after non-heat straightening (A) and after heat straightening (B).

In this case, the higher the difference (delta) between the starting coloured lock and the final lightened locks, the better the lightening result obtained. Again, the locks treated with Coloro have shown better performance under both the straightening conditions A and B, than locks treated with Benchmark MEA and Benchmark NH3.

### Example 9 - Colouring coverage of decoloured hair after application of a dye

A starting overall number of 90 locks of yak hair were subjected to a first step of decolouration, and then subj ected to a second step of decolouration but only on the hair ends, as shown in Figure 6A-C, left locks denoted as "starting partially decoloured hair" in order to emphasize that two different decolourations were performed on the same lock.

Each starting lock was then coloured with Nuance 7 (Fig. 6A) or Nuance 6 (Fig. 6B) or Nuance 5 (Fig. 6C), in:
Group I: "Coloro"
Group II: "Benchmark NH3"
Group III: "Benchmark MEA"

Figures 6A, 6B and 6C are pictures showing the results of the colouring coverage of decoloured yak locks after application of a dye, as above mentioned.

The results achieved demonstrate that Benchmark MEA offers a good colouration on the lock portion subjected to a single decolouration for all the Nuances, but show an unpleasant dark metallic colouration on the end portion doubly decoloured.

Benchmark NH3 shows an unsatisfactory colour coverage, with a visually opaque appearance.

Conversely, Coloro has shown an appreciable colour coverage throughout the lock length and a pleasant brilliant appearance.

### Examples 10-11

The Example 8 was repeated wherein, instead of the composition of Example 3, the composition of Example 1 and Example 2 were used, thus giving Group IV and Group V, respectively.

As said, the aim of this test was to investigate the possibility of lighten the initial intense brown colour (Nuance 6) with a further coloration step involving the Nuance 10, so that the higher the difference (delta) between the starting coloured lock and the final lightened locks, the better the lightening result obtained.

It was observed that the locks treated with the composition of Example 1 (i.e. Group IV), as well as the locks treated with composition of Example 2 (i.e. Group V), have shown better performance under both the straightening conditions A and B, even than locks treated with Coloro.

It is believed that the additional presence of aminomethyl propanol, and ammonium bicarbonate in the alkalizing agent, further improve the lightening action of the composition of the invention on dyed hair.

## Claims

1. A hair-dyeing base composition comprising an alkalizing agent, said alkalizing agent comprising 0.01-10.00wt% of dimethylglucamine and 0.01-2.00wt% of ammonia, based on the composition weight, and being free of monoethanolamine.

2. The composition of claim 1, wherein the weight ratio between dimethylglucamine and ammonia is 1:1 to 1:5, preferably 1:1.1 to 1:3.5, more preferably 1:1.2 to 1:2.5.

3. The composition of claim 1 or 2, said alkalizing agent comprises 0.05-5.00wt% of dimethylglucamine and 0.30-1.90wt% of ammonia, based on the composition weight, preferably, 0.50-2.00wt% of dimethylglucamine and 0.50-1.85wt% of ammonia.

4. The composition of any one claims 1-3, wherein said alkalizing agent further comprises aminomethyl propanol, ammonium bicarbonate, or a mixture thereof.

5. The composition of claim 4, wherein said alkalizing agent further comprises a mixture of 0.01-3.00wt% of aminomethyl propanol, 0.01-2.50wt% of ammonium bicarbonate, based on the composition weight, preferably, a mixture of 0.10-2.90wt% of aminomethyl propanol, 0.10-2.00wt% of ammonium bicarbonate.

6. The composition of claim 4 or 5, wherein said alkalizing agent comprises a mixture of 0.95-1.90wt% of dimethylglucamine, 1.00-1.80wt% of ammonia, 0.50-2.85wt% of aminomethyl propanol, and 0.50-1.50wt% of ammonium bicarbonate, based on the composition weight.

7. The composition of any one claims 4-6, wherein said alkalizing agent consists of a mixture of dimethylglucamine, ammonia, aminomethyl propanol, and ammonium bicarbonate.

8. The composition of any one claims 1-7, said composition being free of ethoxylated compounds, polyethylene glycols, alkylbenzene sulfonates, and silicones.

9. The composition of any one claims 1-8, said composition further comprising water, at least a primary intermediate, at least a secondary intermediate, at least an antioxidant, a lipophilic matrix, and a hydrophilic matrix.

10. A multi-compartment kit for dyeing hair, the kit comprising a first compartment A) comprising the hair-dyeing base composition of any one claims 1-9, and a dye, and a second compartment B) comprising an oxidizing component.

11. The multi-compartment kit of claim 10, wherein the oxidizing component comprises oxygen peroxide.

12. Use of the hair-dyeing base composition of any one claims 1-9, or use of the multi-compartment kit of claim 10 or 11, for permanently dyeing hair.

13. A hair-dyeing method comprising the steps of:
i) mixing the hair-dyeing base composition of any one of claims 1-9, with a dye and an oxidizing agent, until a homogeneous cream is obtained;
ii) applying the cream to hair; and
iii) rinsing with water and washing with a post-colouring shampoo.

14. The hair-dyeing method of claim 13, wherein in step ii), the cream is left on hair for 20-50 minutes, before rinsing with water.

15. A process of producing the hair-dyeing base composition of any one of claims 1-9, said process comprising the steps of:
a) preparing a hydrophilic phase, by mixing hydrophilic ingredients and the alkalizing agent of the invention, at 70-75°C;
b) preparing a lipophilic phase, by mixing lipophilic ingredients at 70-75°C;
c) adding the lipophilic phase to the hydrophilic phase, under stirring, until a homogenous emulsion is obtained; and
d) the emulsion is kept under stirring for a few minutes and then cooled below 50°C, thus achieving the hair-dyeing base composition.
